**Europäisches Patentamt**

**European Patent Office**

**Office européen des brevets**

(19)

(11) Veröffentlichungsnummer : **0 092 742 B2**

# (12) NEUE EUROPÄISCHE PATENTSCHRIFT

(45) Veröffentlichungstag der neuen Patentschrift :
**09.12.92 Patentblatt 92/50**

(51) Int. Cl.⁵ : **F24F 3/16,** A61N 1/44, H01T 23/00

(21) Anmeldenummer : **83103607.4**

(22) Anmeldetag : **14.04.83**

(54) **Vorrichtung zum Ionisieren eines Fluids.**

(30) Priorität : **21.04.82 CH 2409/82**

(43) Veröffentlichungstag der Anmeldung :
**02.11.83 Patentblatt 83/44**

(45) Bekanntmachung des Hinweises auf die
Patenterteilung :
**13.11.85 Patentblatt 85/46**

(45) Bekanntmachung des Hinweises auf die
Entscheidung über den Einspruch :
**09.12.92 Patentblatt 92/50**

(84) Benannte Vertragsstaaten :
**AT DE FR GB IT SE**

(56) Entgegenhaltungen :
**DE-A- 2 363 284**

(56) Entgegenhaltungen :
**DE-A- 2 449 227**
**DE-A- 3 109 528**
**DE-U- 1 857 207**
**FR-A- 2 262 424**
**GB-A- 1 059 417**
**SU-A- 171 110**
**US-A- 4 227 894**
**DIN 57470**

(73) Patentinhaber : **Spengler, Walter**
**Strehlgasse 23**
**CH-4105 Biel-Benken (CH)**

(72) Erfinder : **Spengler, Walter**
**Strehlgasse 23**
**CH-4105 Biel-Benken (CH)**

(74) Vertreter : **Hofmann, Rolf L.**
**Patmed AG Innere Margarethenstrasse 15**
**CH-4010 Basel (CH)**

EP 0 092 742 B2

## Description

La présente invention a pour objet un dispositif pour l'ionisation d'un fluide tel que de l'air utilisé pour le conditionnement d'un espace ou d'une zone à climatiser par ionisation ou bien pour décharger l'électricité statique et du type dans lequel le fluide est soumis dans une enceinte de climatisation à l'action d'au moins un organe d'ionisation, tel qu'un jeu d'électrodes, avant d'être déchargé dans l'espace à climatiser par ionisation.

Les dispositifs d'ionisation de type connu comportent généralement des jeux d'électrodes portées à une tension électrique alternative ou continue relativement élevée (par exemple comprise entre 2 et 5 kV pour les installations à débit d'air important). Ces électrodes sont disposées selon des réseaux géométriques dans les conduites ou canaux parcourus par le gaz ou l'air à ioniser et se présentent sous forme de pointes ou de fils portés par les parois de ces conduites ou canaux. Ces dispositifs d'ionisation donnent relativement satisfaction dans les installations industrielles de traitement de gaz par ionisation telles que les installations de dépoussiérage par précipitation électrostatique mais se révèlent beaucoup mois efficaces dans les installations de climatisation d'air par ionisation.

Ces installations de taille plus réduite sont, par exemple, destinées à supprimer l'électricité statique qui apparaît sur les fibres textiles en cours de trituration ou dans les équipements électroniques sensibles tels que les ordinateurs ou les circuits intégrés, en particulier au cours de leur réalisation et de leur assemblage. Ce manque d'efficacité des installations d'ionisation de taille moyenne paraît tenir au fait que, pour des raisons de coût et d'organisation de l'installation de climatisation, on installe les électrodes d'ionisation dans une enceinte de traitement de l'air et que l'on distribue l'air traité par des conduites relativement longues dans des zones d'utilisation telles que le voisinage de nappes de fils textiles chargés d'électricité statique ou bien de tables d'assemblage de circuits imprimés.

Après ionisation positive ou négative de l'air de traitement, le parcours relativement turbulent de cet air ionisé dans les conduites de distribution provoque une "recombinaison" des ions positifs et négatifs précédemment dissociés et cette "recombinaison" ou dépolarisation de l'air réduit considérablement le taux d'ionisation de l'air traité lorsqu'il est délivré dans la zone d'utilisation constituant un espace à climatiser par ionisation.

Pour diminuer la recombinaison des ions dans GB-A-1 059 417 et DE-A-2 449 227, il a déjà été proposé de disposer l'organe d'ionisation à proximité immédiate de la sortie de décharge à l'intérieur d'un canal de décharge d'air ionisé mais l'expérience montre que les ions se recombinent à la traversée des vannes de décharge ou des dispositifs de protection que l'on est obligé de disposer autour des électrodes constituant d'organe d'ionisation.

Selon GB-A-1 059 417, l'ionisation de l'air peut en outre être réalisée par des corps émetteurs de radiations qui sont fixés sur les pales d'une hélice de soufflage à l'air libre. Les risques de recombinaison au cours de la décharge de l'air sont ainsi radicalement réduits mais un tel appareillage ne peut s'appliquer à des installations de soufflage à jets ou à organe de soufflage central.

Par ailleurs, on propose dans DE-A-2 363 284 un dispositif d'ionisation formant source de plasma et dans lequel des électrodes positives et négatives traversent axialement un passage de sortie pour venir pointer juste à la sortie du passage formant canal de décharge. Un tel dispositif place certes les électrodes à la sortie du canal de décharge en évitant la recombinaison de ions du plasma mais il laisse ces électrodes exposées aux agressions mécaniques ou aux court-circuits provoqués par les éléments contenus dans l'enceinte de climatisation.

L'un des buts de la présente invention est précisément de supprimer presque complètement l'inconvénient de la recombinaison des ions du fluide tel que de l'air traité par une opération d'ionisation, sans augmenter de façon importante le coût des appareillages de traitement par ionisation, en utilisant une installation centrale de soufflage et tout en isolant mécaniquement les électrodes de l'enceinte de climatisation qui peut contenir des éléments agressifs à l'égard des électrodes.

A cet effet, lorsque la décharge de l'enceinte de climatisation s'effectue par une pluralité de sorties à proximité de chacune desquelles est disposé au moins un organe d'ionisation, afin de réduire les possibilités de recombinaison et/ou de dépolarisation des ions générés par l'organe d'ionisation, cet organe d'ionisation est placé dans l'espace à climatiser, à l'extérieur de chacun des canaux de décharge de l'enceinte de climatisation, et est séparé de l'espace à climatiser par des parois perforées d'une pluralité de passages d'une chambre de distribution, à partir de laquelle le fluide est déchargé dans l'espace à climatiser par une pluralité de sorties formées par lesdits passages et telles que celles d'une pomme d'arrosoir.

L'organe d'ionisation est alors placé, de préférence, sensiblement au centre de la chambre de distribution et, en variante, les parois perforées de la chambre de distribution sont aménagées pour constituer une protection à l'égard de l'organe d'ionisation si celui-ci présente des dangers d'exploitation, tels que des dangers électriques pour des électrodes ou des dangers d'irradiation pour une source radio-active.

Selon encore un autre mode de réalisation de l'invention, lorsque des moyens de réduction de la turbulence sont disposés dans l'enceinte de climatisation à proximité de l'organe d'ionisation, ces moyens de réduction de la turbulence sont disposés à l'amont immédiat de l'organe de climatisation dans le sens d'écoulement du fluide.

Les moyens de réduction de la turbulence peuvent être constitués par les électrodes de l'organe d'ionisation, ces électrodes étant disposées sous forme de lames ou de fils.

En variante, les moyens de réduction de la turbulence sont constitués par la disposition selon une forme aplatie de la section de décharge de l'enceinte de climatisation afin d'orienter le flux d'air sous la forme d'une lame relativement mince avant son passage devant l'organe d'ionisation. De même, le ou les canaux de décharge de l'enceinte de climatisation peuvent présenter une forme convergente en direction de l'organe d'ionisation, de manière à bien centrer sur l'organe d'ionisation, tel qu'au moins une électrode, le jet d'air à ioniser ou à désioniser.

D'autres buts, avantages et caractéristiques de l'invention apparaîtront à la lecture de la description de divers modes de réalisation de l'invention, faite à titre non limitatif et en regard du dessin annexé où:

- la figure 1 est une représentation schématique d'une installation d'ionisation selon l'état de la technique avec un schéma synoptique de la partie d'excitation électrique;
- la figure 2 représente la version modifiée selon l'invention de l'installation de la figure 1;
- la figure 3 représente en coupe schématique une forme de réalisation de l'invention applicable à une distribution d'air ionisé existante dotée d'un système de répartition en pluie;
- la figure 4 représente schématiquement une application de l'invention à une installation d'ionisation à grand débit d'air soufflé sur une section réduite.

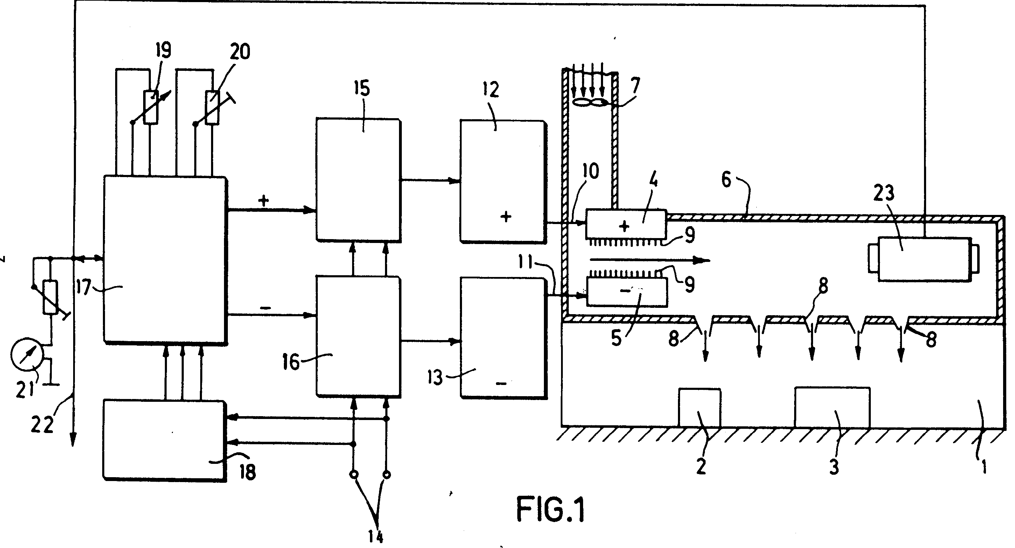

L'installation d'ionisation de l'air de type connu et représentée sur la figure 1, vise à augmenter la teneur de l'air déchargé dans un local à climatiser 1 en ions négatifs ou positifs selon les besoins de décharge électrostatique de machines ou d'appareillages 2, 3, disposés dans ce local. Pour celà, des électrodes positives 4 et négatives 5 sont disposées dans une canalisation d'air 6 ans laquelle de l'air extérieur préalablement filtré est soufflé par un ventilateur 7 et délivré dans le local 1 par une pluralité de buches ou d'ouvertures de sortie 8. Pour augmenter leur efficacité électrique, les électrodes 4 et 5 réalisées sous forme d'une ou plusieurs plaques, sont équipées de pointes 9 qui constituent des zones de concentration du gradient de potentiel électrique dans l'air à traiter et à partir desquelles se forment les ions qui se répartissent dans l'air à ioniser distribué par les ouvertures 8.

Les électrodes 4 et 5 sont reliées par l'intermédiaire de câbles 10 et respectivement 11 à des transformateurs 12 et 13 générateurs de haute tension alternative ou continue (dans ce dernier cas, des redresseurs non représentés sont intercalés). Les transformateurs 12 et 13 sont reliés aux phases 14 d'un réseau électrique par l'intermédiaire de contrôleurs de phase 15 et 16 contrôlés par une électronique de réglage 17 reliée au réseau électrique en 18 et soumise à un réglage de valeur de consigne 19 via un amplificateur 20. L'électronique de réglage 17 est reliée à un indicateur de champ électrique 21, lui-même relié, le cas échéant, à un enregistreur de champ électrique par un câblage 22. Un indicateur d'ionisation 23 placé dans la canalisation 6 peut fournir une indication de contre-réaction à l'électronique de réglage 17 qui pilote, selon les besoins, la tension et le sens de la tension des électrodes de réglage 4 et 5.

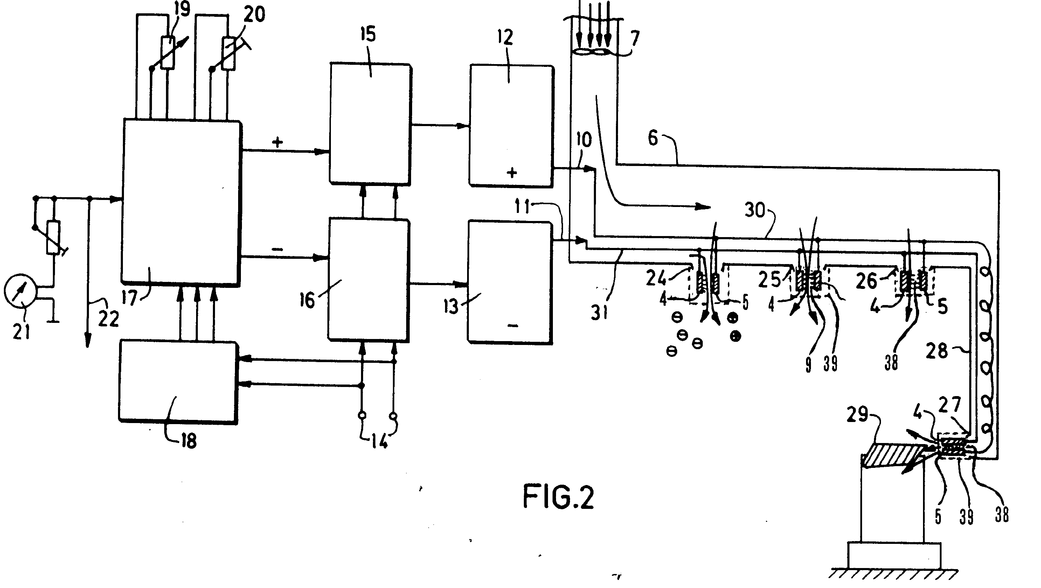

Si l'on se reporte au mode de réalisation de l'invention représenté sur la figure 2, où les éléments identiques à ceux de la figure 1 présentent les mêmes références, on voit que les électrodes positives 4 et négatives 5 qui étaient disposées en une zone unique de la canalisation 6 très en amont des ouvertures de sortie 8 de l'air, sont reportées juste à la sortie de chacune des sorties de décharge légèrement convergentes 24, 25, 26, 27 de la canalisation 6, en dehors de cette canalisation et déjà dans le local à climatiser. Pour celà, les câbles à haute tension 10 et 11 sont prolongés à l'intérieur ou à l'extérieur de la canalisation 6 et sont reliés à des plaques d'électrodes 4, respectivement 5, munies le cas échéant, de pointes 9 et présentant généralement de plus faibles dimensions que les plaques d'électrodes 4 et 5 de la réalisation de la figure 1.

La canalisation 6 peut être prolongée par une conduite 28 de plus petit diamètre alimentant la sortie 27 pour desservir par exemple un poste de décharge de l'électricité statique d'une nappe de fils textiles 29. Le nombre d'électrodes est multiplié par le nombre de sorties de décharge qu'il y a évidemment intérêt à réduire dans la réalisation de l'invention, mais l'équipement de contrôle et de génération de la haute tension d'ionisation reste identique à celui de la figure 1 selon l'état de la technique. Comme on peut aisément l'imaginer en considérant la figure 2, les possibilités de "recombinaison" des ions positifs et négatifs dans la conduite 6 sous l'effet des turbulences et des chocs contre les parois de cette conduite sont radicalement éliminés et le rendement du traitement d'ionisation est considérablement accru dans un rapport tel qu'il compense largement l'augmentation du nombre d'électrodes et la nécessité de tirer des câbles à haute tension ou des conducteurs isolés 30 et 31 à partir des câbles de sortie 10 et 11 de l'équipement de génération de la haute tension.

Dans les solutions connues, les électrodes sont logées à l'intérieur d'une courte tubulure d'une sortie de décharge et cette disposition présente l'avantage de protéger les électrodes des risques de court circuit accidentel. Selon l'invention, on désire à la fois réduire radicalement les risques de recombinaison des ions et procéder à leur diffusion rapide dans l'ambiance à climatiser. On peut, pour cela, placer les électrodes 4 et 5, par exemple sous forme de nappes de fils, juste à la sortie des tubulures de décharge de la canalisation d'alimentation 6 constituant une enceinte de climatisation. On a représenté sur la figure 2 une solution où les électrodes 4 et 5 sont placées dans l'espace à climatiser juste à la sortie des canaux de sortie 24-27 de forme convergente, de telle manière que l'espace intercalaire 38 entre les pointes 9 soit balayé par le jet d'air qui s'échappe des sorties pour se diluer dans l'atmosphère de l'espace à climatiser. Lorsque les électrodes 4, 5 présentent des dangers de court circuit ou d'électrocution, on peut les entourer d'une paroi ou d'une cage ou grille de protection 39 perforée d'une multitude de passages. L'indicateur d'ionisation 23 fournissant une contre-réaction à l'électronique de réglage 17 est, soit supprimé, soit placé au voisinage d'une sortie se décharge au-delà des électrodes 4, 5.

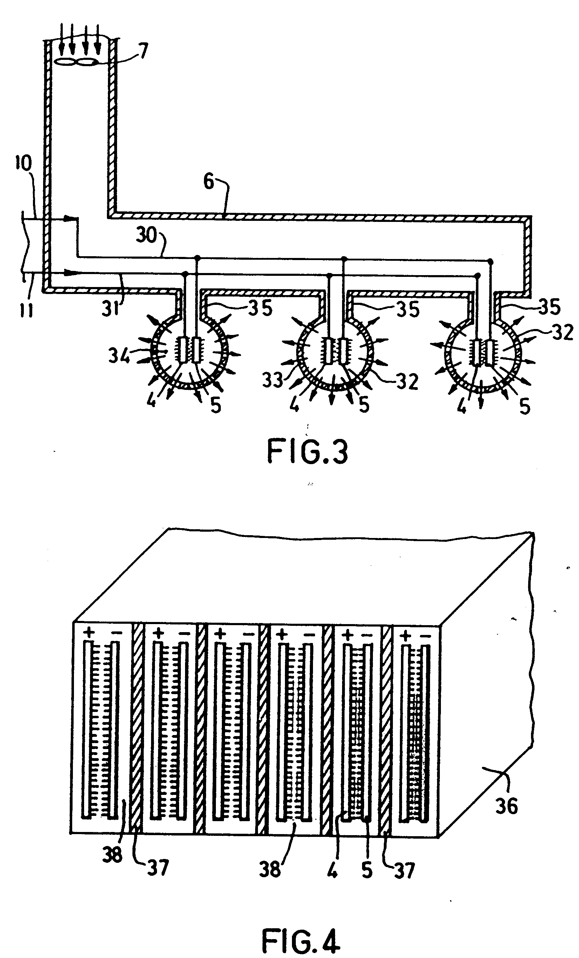

Dans le mode de réalisation représenté sur la figure 3, on voit seulement la disposition des électrodes d'ionisation 4 et 5 par rapport aux canalisations et conduites de distribution de l'air ou du flux en général. On suppose qu'il s'agit d'une installation de distribution d'air en pluie à partir de têtes de distribution en forme de pomme d'arrosoir 32 branchées sur la canalisation 6 pour distribuer l'air dans toutes les directions par une multitude de petits trous 33 percés dans leur paroi.

Pour équiper une telle installation de distribution d'air d'un système d'ionisation de l'air selon l'invention, on ne peut bien sûr pas équiper chaque trou de sortie d'un jeu d'électrodes d'ionisation extérieures. Les électrodes d'ionisation 4 et 5 reliées aux câbles à haute tension 10 et respectivement 11 de l'installation de génération et de contrôle de la haute tension via un jeu de barres 30 et 31, sont disposées, sensiblement au centre de la chambre de distribution 34 constituée à l'intérieur de chaque pomme d'arrosage 32, en aval de la conduite d'amenée 35 à cette pomme 32, cette conduite 35 constituant en fait un canal de décharge de l'enceinte de climatisation 6. Les possibilités de recombinaison des ions sont augmentées par rapport à la solution de la figure 2, mais néanmoins réduites par rapport à l'état de la technique représenté sur la figure 1, car il est possible de réaliser la pomme 32 en un matériau tel que de la matière plastique, ne provoquant que peu de »recombinaisons«.

Le mode de réalisation représenté sur la figure 4 s'applique à une distribution d'air ionisé de grande section dont seul le caisson de distribution 36 est représenté en perspective.

Selon l'invention, on subdivise par des cloisons 37 le caisson 36 en une pluralité de sorties 38 de section aplatie et l'on dispose en face de chacune de ces sorties plates 38 un jeu d'électrodes d'ionisation 4 et 5. A cause de la forme aplatie des sections de sortie 38, la turbulence de l'air est réduite et les possibilités de »recombinaison« et de dépolarisation des ions produits par les électrodes 4 et 5 sont réduites après la sortie du caisson 36 qui assure un grand débit de décharge d'air.

Pour réduire la turbulence dans les modes de réalisation des figures 2 et 3, on dispose des plaques ou des filtres dans la canalisation 6 à l'amont immédiat des électrodes d'ionisation 4 et 5. Un autre moyen de réduire la turbulence de l'air à la traversée des électrodes d'ionisation 4 et 5 et après les sorties de décharge dans l'espace à climatiser par ionisation, consiste à réaliser les électrodes 4 et 5 sous forme de lames disposées dans le sens d'écoulement du flux d'air en face des sorties de la canalisation 6 constituant une enceinte de climatisation.

On doit comprendre que l'organe d'ionisation constitué par les électrodes 4 et 5 peut être remplacé par des charges électriques obtenues par frottement sur un corps isolant ou par un corps radioactif qui peut remplir en même temps une fonction de stérilisation du fluide déchargé par les sorties.

**Revendications**

1. Dispositif pour l'ionisation d'un fluide tel que l'air utilisé pour le conditionnement d'un espace (1) ou d'une zone à climatiser par ionisation ou bien pour décharger l'électricité statique et du type dans lequel le fluide est soumis dans une enceinte de climatisation à l'action d'au moins un organe d'ionisation tel qu'un jeu d'électrodes (4, 5) avant d'être déchargé dans l'espace (1) à climatiser par ionisation, la décharge de l'enceinte de climatisation s'effectuant par une pluralité de sorties (35) à proximité de chacune desquelles est disposé au moins un organe d'ionisation (4, 5) afin de réduire les possibilités de recombinaison et/ou de dépolarisation des ions générés par l'organe d'ionisation (4, 5) caractérisé en ce que l'organe d'ionisation est placé dans l'espace à climatiser à l'extérieur de chacun des canaux (24 à 27, 35) de décharge de l'enceinte de climatisation, et en ce que l'organe d'ionisation (4, 5) est séparé de l'espace à climatiser (1) par des parois (39, 32) perforées d'une pluralité de passages d'une chambre de distribution (34), à partir de laquelle le fluide est déchargé dans l'espace à climatiser (1) par une pluralité de sorties (33) formées par lesdits passages et telles que celles d'une pomme d'arrosoir.

2. Dispositif selon la revendication 1, caractérisé en ce que l'organe d'ionisation (4, 5) est placé sensiblement au centre de la chambre de distribution (34).

3. Dispositif selon la revendication 1 ou 2, caractérisé en ce que les parois perforées (39, 32) de la chambre de distribution (34) sont aménagées pour constituer une protection à l'égard de l'organe d'ionisation (4, 5) si celui-ci présente des dangers d'exploitation tels que des dangers électriques pour des électrodes ou des dangers d'irritation pour une source radio-active.

4. Dispositif selon l'une des revendications 1 à 3, dans lequel des moyens de réduction de la turbulence sont disposés dans l'enceinte de climatisation à proximité de l'organe d'ionisation (4, 5), caractérisé en ce que les moyens de réduction de la turbulence sont disposés à l'amont immédiat de l'organe d'ionisation (4, 5) dans le sens d'écoulement du fluide.

5. Dispositif selon la revendication 4, caractérisé en ce que les moyens de réduction de la turbulence sont constitués par la disposition selon une forme aplatie de la section de décharge (38) de l'enceinte de climatisation afin d'orienter le flux d'air sous la forme d'une lame relativement mince avant son passage devant l'organe d'ionisation (4, 5).

6. Dispositif selon l'une des revendications 1 à 5, caractérisé en ce que le ou les canaux(24 à 27, 35) de décharge de l'enceinte de climatisation présentent une forme convergente en direction de l'organe d'ionisation (4, 5).

**Claims**

1. Device for ionizing a fluid such as air used for conditioning a space (1) or an area to be climatized by ionization or for discharging static electricity and of the type in which the fluid is subjected, in a climatization enclosure, to the action of at least one ionization unit such as a set of electrodes (4, 5) before being discharged into the space (1) to be climatized by ionization, discharge of the climatization enclosure being effected through a plurality of outlets (35) in the proximity of each one of which at least one ionization unit (4, 5) is arranged in order to reduce the possibilities of recombination and/or depolarization of the ions generated by the ionization unit (4, 5), characterized in that the ionization unit is located in the space to be climatized externally of each one or the discharge channels (24 to 27, 35) of the climatization enclosure and in that the ionization unit (4, 5) is separated from the space to be climatized (1) by walls (39, 32), said walls being perforated with a plurality of passages from a distribution chamber (34) from which said fluid is discharged into the space to be climatized (1) through a plurality of outlets (33) formed by said passages and arranged similarly to the outlets of a sprinkler head.

2. Device according to claim 1, characterized in that said ionization unit (4, 5) is located substantially at the center of said distribution chamber (34).

3. Device according to claim 1 or 2, characterized in that said perforated walls (39, 32) of the distribution chamber (34) are adapted so as to provide protection against said ionization unit (4, 5) if the latter suffers from operational dangers such as electrical danger concerning itselectrodes or radiation danger for a radioactive source.

4. Device according to any one of claims 1 to 3, in which turbulence-reducing means are provided in the climatization enclosure close to the ionization unit (4, 5), characterized in that said turbulence-reducing means are arranged immediately upstream of said ionization unit (4, 5) in the sense of flow of said fluid.

5. Device according to claim 4, characterized in that said turbulence-reducing means are constituted by an arrangement in which the discharge section (38) and the climatization enclosure have a flattened form whereby the, airflow is oriented in the form of a relatively thin lamina flow prior to passage thereof in front of said ionization unit (4, 5).

6. Device according to one of claims 1 to 5, characterized in that the discharge channel or channels (24 to 27, 35) of the climatization enclosure are of a shape which converges in the direction of said ionization unit (4, 5).

**Patentansprüche**

1. Vorrichtung zur Ionisation eines Mediums, wie Luft, zum Konditionieren eines Raumes (1) oder einer zu klimatisierenden Zone durch Ionisation, oder zur Ableitung statischer Elektrizität, in welcher das Medium in einer Klimatisierkammer der Wirkung wenigstens eines Ionisationsorgans, wie einem Elektrodensatz (4, 5) unterworfen wird, bevor es in den durch Ionisation zu klimati-sierenden Raum (1) abgeleitet wird, wobei die Ab-leitung von der Klimatisierkammer durch eine Mehrzahl von Auslässen (8) erfolgt, in deren Nä-he jeweils wenigstens ein Ionisationsorgan (4, 5) angeordnet ist, um das Risiko der Rekombination und/oder Depolarisation der durch das Ionisati-onsorgan (4, 5) erzeugten Ionen zu vermindern, dadurch gekennzeichnet, dass das Ionisationsor-gan innerhalb des zu klimatisierenden Raumes außerhalb jeder Ableitungsleitung (24 bis 27, 35) der Klimatisierkammer angeordnet ist und dass das Ionisationsorgan (4, 5) von dem zu klimatisierenden Raum (1) durch unter Ausbildung einer Mehrzahl von Durchlässen perforierte Trennwandungen (39, 32) einer Verteilerkammer (34) getrennt ist, aus welcher das Medium in den zu klimatisierenden Raum (1) durch eine Mehrzahl von Auslässen (33) ausgegeben wird, die von den Durchlässen gebildet werden und denen eines Brausekopfes ähneln.

2. Vorrichtung nach Anspruch 1, dadurch gekennzeichnet, dass das Ionisationsorgan (4, 5) im Wesentlichen im Zentrum der Verteilerkammer (34) angeordnet ist.

3. Vorrichtung nach Anspruch 1 oder 2, dadurch gekennzeichnet, dass die perforierten Trennwandungen (39, 32) der Verteilerkammer (34) derart ausgebildet sind, dass sie einen Schutz für das Ionisationsorgan (4, 5) bilden, wenn beim letzteren Betriebsgefahren bestehen, wie elektrische Gefährdung der Elektroden oder Bestrahlungsgefahr einer Quelle radioaktiver Strahlung.

4. Vorrichtung nach einem der Ansprüche 1 bis 3, dadurch gekennzeichnet, dass Turbulenzverminderungsmittel in der Klimatisierkammer in Nähe des Ionisationsorgans (4, 5) angeordnet sind, und zwar unmittelbar stromaufwärts vom Ionisationsorgan (4, 5) bezogen auf die Strömungsrichtung des Mediums.

5. Vorrichtung nach Anspruch 4, dadurch gekennzeichnet, dass die Turbulenzverminderungsmittel durch die abgeflachte Ausgestaltung des Ableitabschnitts (38) der Klimatisierkammer gebildet sind, derart, dass die Luftströmung in Form einer verhältnismäßig dünnen Schicht vor ihrem Durchgang vor dem Ionisationsorgan (4, 5) orientiert wird.

6. Vorrichtung nach einem der Ansprüche 1 bis 5, dadurch gekennzeichnet, dass die Ableitung bzw. Ableitungen (24 bis 27, 35) der Klimatisierkammer in Richtung zum Ionisierungsorgan (4, 5) eine konvergente Form besitzen.

FIG.1

EP 0 092 742 B2

FIG.2

FIG.3

FIG.4

9